# FASCICULE DE BREVET EUROPEEN

(11) **EP 0 232 199 B1**
(45) Date de publication et mention de la délivrance du brevet: **03.02.1993**
(21) Numéro de dépôt: 87400134.0
(22) Date de dépôt: 20.01.1987
(51) Int. Cl.: C07C 233/75, C07C 233/81, C07C 235/56, A61K 31/16, A61K 31/245, A61K 31/195

(54) **Composés benzamido aromatiques, leur procédé de préparation et leur utilisation en médecine humaine ou vétérinaire et en cosmétique**
Aromatische Benzamido-Verbindungen, Verfahren zu ihrer Herstellung und ihre Verwendung in Human- oder Tiermedizin und in der Kosmetik
Aromatic benzamido compounds, process for their preparation and their use in human or veterinary medicine as well as in cosmetics

(30) Priorité: 21.01.1986 LU 86258
(43) Date de publication de la demande: 12.08.1987
(73) Titulaire: CENTRE INTERNATIONAL DE RECHERCHES DERMATOLOGIQUES GALDERMA - CIRD GALDERMA, F-06560 Valbonne (FR)
(72) Inventeur: Shroot, Braham Villa 35, Chemin de Val Bosquet 06600 Antibes (FR); Eustache, Jacques, F-06130 Grasse (FR); Bernardon, Jean-Michel, 06650 Nice (FR)
(74) Mandataire: Nony, Michel

(56) Documents cités:
- FR-A- 2 296 407
- GB-A- 2 164 648

## Description

La présente invention a pour objet de nouveaux dérivés benzamido aromatiques, leur procédé de préparation et leur utilisation en médecine humaine et vétérinaire et en cosmétique.

Ces nouveaux dérivés benzamido-aromatiques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, ainsi qu'une activité antitumorale. En outre, ces dérivés peuvent être utilisés dans le traitement de l'atopie, qu'elle soit cutanée ou respiratoire et du psoriasis rhumatoïde.

Ils trouvent également une application dans le domaine ophtalmologique, notamment pour le traitement des cornéopathies.

L'état de la technique concernant des composés de ce type est essentiellement matérialisé par la demande de brevet français n° 2.296.407 (75 40257) qui décrit des acide benzoïques substitués ayant une activité hypoglycémiante.

Les dérivés benzamido aromatiques selon l'invention peuvent être représentés par la formule générale suivante : 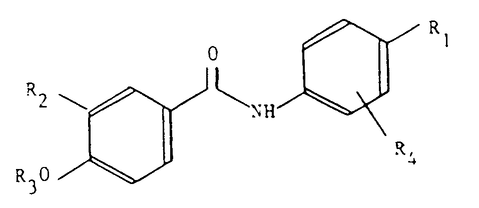
dans laquelle :
R₁ représente - CH₂OH, -CHOHCH₃ ou -COR₅,
R₅ représentant un atome d'hydrogène, un radical alkyle inférieur, le radical -OR₆ 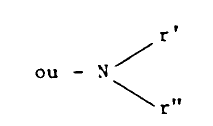
R₆ représentant un atome d'hydrogène, un radical alkyle inférieur ou un radical mono ou polyhydroxyalkyle, r' et r'' représentant un atome d'hydrogène, un radical alkyle inférieur, un radical mono ou polyhydroxyalkyle, un radical aryle ou benzyle éventuellement substitué(s), un reste d'aminoacide ou de sucre aminé ou pris ensemble forment un hétérocycle,
R₂ représente un radical alkyle α,α' disubstitué ayant de 4 à 12 atomes de carbone ou un radical cycloalkyle, mono ou polycyclique ayant de 5 à 12 atomes de carbone dont le carbone de liaison est quaternaire,
R₃ représente un atome d'hydrogène ou un radical alkyle ayant de 1 à 10 atomes de carbone, et
R₄ représente un atome d'hydrogène, un radical alkyle inférieur ou un radical hydroxyle, et les sels desdits dérivés benzamido aromatiques de formule (I) lorsque R₆ représente un atome d'hydrogène.

Lorsque les composés selon l'invention se présentent sous forme de sels, il peut s'agir de sels d'un métal alcalin ou alcalino-terreux ou encore de zinc ou d'une amine organique.

Par radical alkyle inférieur, on doit entendre un radical ayant de 1 à 6 atomes de carbone notamment les radicaux méthyle, éthyle, isopropyle, butyle et tertiobutyle.

Par radical monohydroxyalkyle, on doit entendre un radical ayant 2 ou 3 atomes de carbone, notamment un radical 2-hydroxyéthyle, 2-hydroxypropyle ou 3-hydroxypropyle,

Par radical polyhydroxyalkyle, on doit entendre un radical contenant de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on doit entendre un radical phényle éventuellement substitué par un atome d'halogène, un hydroxyle ou une fonction nitro.

Par radical α,α'-disubstitué ayant de 4 à 12 atomes de carbone, on doit notamment entendre un radical tert-butyle, 1,1-diméthylpropyle, 1-méthyl 1-éthyl propyle, 1-méthyl 1-éthyl hexyle ou 1,1-diméthyl décyle.

Par radical cycloalkyle mono ou polycyclique ayant de 5 à 12 atomes de carbone, dont le carbone de liaison est quaternaire, on doit entendre un radical 1-méthyl cyclohexyle ou 1-adamantyle.

Par reste d'aminoacide, on doit entendre un reste dérivant par exemple de la lysine ou de la glycine.

Par reste d'un sucre aminé, on doit entendre un reste dérivant par exemple de glucosamine, de galactosamine ou de mannosamine.

Lorsque les radicaux r' et r'' pris ensemble forment un hétérocycle, celui-ci est de préférence un radical pipéridino, pipérazino, morpholino, pyrrolidino ou (2-hydroxyéthyl)-4-pipérazino.

Parmi les dérivés benzamido aromatiques de formule (I) ci-dessus préférés, on peut notamment citer les suivants :
- l'acide 4-[3-(1-adamantyl)-4-méthoxybenzamido]benzoïque,
- le 4-[3-(1-adamantyl)-4-méthoxybenzamido]benzoate de méthyle,
- le N-éthyl 4-[3-(1-adamantyl)-4-méthoxybenzamido]benzamide,
- l'acide 4-[3-(1-adamantyl)-4-hydroxybenzamido]benzoïque,
- le 4-[3-(1-adamantyl)-4-hydroxybenzamido]benzoate de méthyle,
- l'acide 4-[3-(1-méthylcyclohexyl)-4-méthoxybenzamido]benzoïque,
- le 4-[3-(1-méthylcyclohexyl)-4-méthoxybenzamido]benzoate d'éthyle,
- l'acide 4-[3-(1-adamantyl)-4-décyloxybenzamido] benzoïque,
- le 4-[3-(1-adamantyl)-4-décyloxybenzamido]benzoate de méthyle,
- l'acide 4-[3-(1-adamantyl)-4-hexyloxybenzamido]benzoïque
- le 4-[3-(1-adamantyl)-4-hexyloxybenzamido]benzoate de méthyle,
- l'acide 4-[3-(1,1-diméthyldécyl)-4-méthoxybenzamido] benzoïque,
- le 4-[3-(1,1-diméthyldécyl)-4-méthoxybenzamido]benzoate de méthyle
- l'acide 4-(3-tert-butyl-4 méthoxybenzamido) benzoïque,
- le 4-(3-tert-butyl-4-méthoxybenzamido)benzoate de méthyle,
- la N-[4-[3-(1-adamantyl)-4-méthoxybenzamido]-benzoyl] pyrrolidine,
- la N-[4-[3-(1-adamantyl)-4-métboxybenzamido]-benzoyl] pipéridine,
- le morpholide de l'acide 4-[3-(1-adamantyl) -4-méthoxybenzamido]benzoïque,
- l'amide tert-butylique de l'acide 4-[3-(1-adamantyl)-4-méthoxybenzamido]benzoïque,
- l'amide éthylique de l'acide 4-[3-(1-adamantyl)-4-méthoxybenzamido]benzoïque,
- L'anilide de l'acide 4-[3-(1-adamantyl)-4-méthoxybenzamido]benzoïque,
- l'amide benzylique de l'acide 4-[3-(1-adamantyl)-4-méthoxybenzamido]benzoïque,
- le 4-[3-(1-adamantyl)-4-méthoxybenzamido]benzoate de 2-hydroxyéthyle,
- le N-(4-acétylphényl)-3-(1-adamantyl)-4-méthoxy benzamide,
- le N-[4-(1-hydroxyéthyl)phényl]-3-(1-adamantyl)-4-méthoxy benzamide,
- l'amide 2-hydroxyéthylique de l'acide 4-[3-(1-adamantyl) -4-méthoxybenzamido]benzoïque,
- le 2-hydroxy-4-[3-(1-adamantyl)-4-méthoxybenzamido]benzoate de méthyle.

La présente invention a également pour objet le procédé de préparation des composés de formule (I) selon le schéma réactionnel suivant : 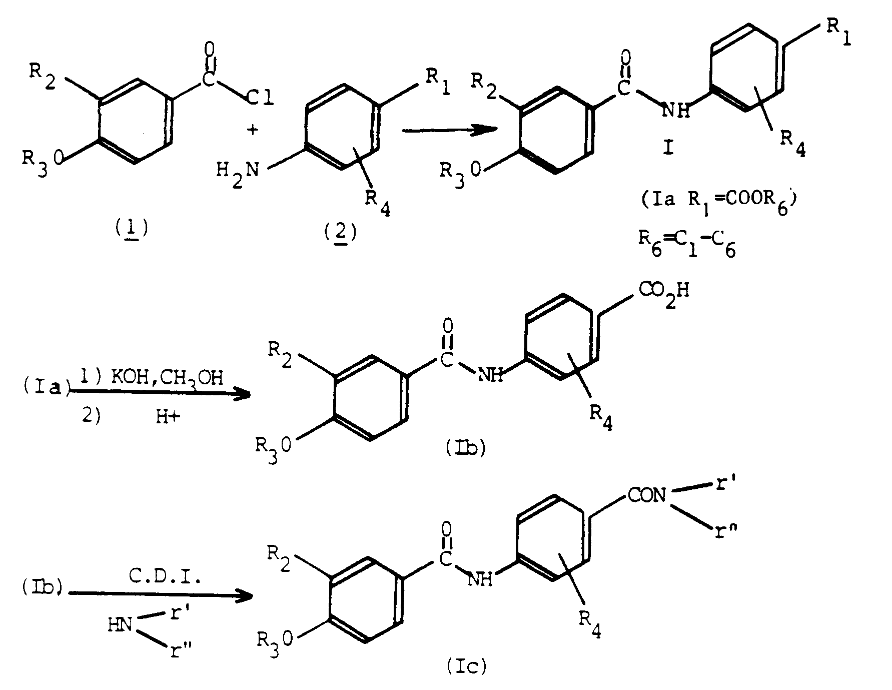
L'étape principale de cette préparation consiste à faire réagir en milieu anhydre, dans un solvant organique de préférence le tétrahydrofuranne et en présence d'une amine tertiaire, une forme activée d'un acide benzoïque substitué, par exemple un chlorure d'acide (1) sur un composé aminé de formule (2), la réaction étant conduite à température ambiante et sous agitation.

A partir de l'ester (Ia) on accède par saponification à l'acide correspondant (Ib) qui peut ensuite être activé, par exemple à l'aide de N,N'-carbonyldiimidazole (CDI) ou par conversion en chlorure d'acide, et transformé en amide de formule (Ic), par action d'une amine de formule 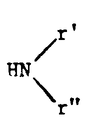
(r' et r'' ayant les mêmes significations que données ci-dessus).

Lorsque R₆ représente un radical monohydroxy ou polyhydroxyalkyle il est préférable de préparer l'acide (Ib) à partir de l'ester méthylique (Ia) (R₆ = -CH₃) et ensuite d'estérifier l'acide ainsi obtenu en ester de l'alcool mono ou polyhydrique choisi selon les méthodes connues.

Les composés dans lesquels R₁=-CH₂OH et -CHOH-CH₃ sont obtenus de façon conventionnelle par réduction respectivement des esters et cétones correspondants.

La présente invention a également pour objet à titre de médicament les composés de formule (I) telle que définie ci-dessus.

Ces composés présentent une excellente activité dans le test d'inhibition de l'ornithine décarboxylase après induction, par "tape stripping", chez le rat nu (M. Bouclier et al, DERMATOLOGICA 169 n°4 1984). Ce test est admis comme mesure de l'action inhibitrice de certains composés sur les phénomènes de prolifération cellulaire.

Ces composés conviennent particulièrement bien pour traiter les affections dermatologiques liées à un désordre de la kératinisation (différenciation-prolifération) ainsi que les affections dermatologiques, ou autres, à composante inflammatoire et/ou immunoallergique notamment :
- les acnés vulgaires, comédoniennes ou polymorphes, les acnés séniles, solaires et les acnés médicamenteuses ou professionnelles,
- les formes étendues et/ou sévères de psoriasis, et les autres troubles de la kératinisation, et notamment les ichtyoses et les états ichtyosiformes,
- la maladie de Darier
- les kératodermies palmo-plantaires
- les leucoplasies et états leucoplasiformes, le lichen plan
- toutes proliférations dermatologiques bénignes ou malignes, sévères ou étendues.

Ils sont également actifs dans le traitement des tumeurs, du psoriasis rhumatoïde, des atopies cutanées ou respiratoires ainsi que dans le traitement de certains problèmes ophtalmologiques relatifs aux cornéopathies.

La présente invention a donc également pour objet des compositions médicamenteuses contenant au moins un composé de formule (I) tel que défini ci-dessus, ou un de ses sels.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels.

Les composés selon l'invention présentent une bonne stabilité à la lumière et à l'oxygène.

Les composés selon l'invention sont généralement administrés à une dose journalière d'environ 0,01mg/Kg à 5mg/Kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, des sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse selon l'indication clinique.

Par voie oculaire, ce sont principalement des collyres.

Ces compositions contiennent au moins un composé de formule (I) tel que défini ci-dessus ou un de ses sels, à une concentration de préférence comprise entre 0,0001 et 5% par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme de lotion, gel, savon ou shampooing.

La concentration en composé de formule (I) dans les compositions cosmétiques, est comprise entre 0,0001 et 0,1% en poids et de préférence entre 0,001 et 0,01% en poids.

Les compositions médicamenteuses et cosmétiques selon l'invention peuvent contenir des additifs inertes ou même pharmacodynamiquement ou cosmétiquement actifs et notamment : des agents hydratants comme la thiamorpholinone et ses dérivés ou l'urée ; des agents antiséborrhéiques ou antiacnéiques, tels que la S-carboxyméthylcystéine, la S-benzyl-cystéamine, leurs sels et leurs dérivés, la tioxolone ou le peroxyde de benzoyle ; des antibiotiques comme l'érythromycine et ses esters, la néomycine, les tétracyclines ou les polyméthylène-4,5 isothiazolinones-3 ; des agents favorisant la repousse des cheveux, comme le "Minoxidil" (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphénylimidazolidine 2,4-dione) ; des agents anti-inflammatoires stéroïdiens et non stéroïdiens ; des caroténoïdes et, notamment le ß-carotène ; des agents anti-psoriasiques tels que l'anthraline et ses dérivés et les acides eicosatétraynoïque-5,8,11,14 et triynoïque-5,8,11, leurs esters et leurs amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que de 1'α-tocophérol, le butylhydroxyanisole ou le butylhydroxy toluène.

On va maintenant donner, à titre d'illustration et sans aucun caractère limitatif, plusieurs exemples de préparation des composés actifs de formule (I) selon l'invention ainsi que des exemples de compositions les contenant.

### EXEMPLE 1

### 4-[3-(1-adamantyl)-4-méthoxybenzamido]benzoate de méthyle

### (a) Acide 3-(1-adamantyl)-4-méthoxybenzoïque.

Dans un ballon, on introduit 5,4g (225 mmoles) de Mg et 30ml de tétrahydrofuranne. On ajoute goutte à goutte une solution de 48,3 g (150 mmoles) de 2-adamantyl-4 bromo anisole, 6ml (70 mmoles) de dibromoéthane dans 300ml de tétrahydrofuranne. On chauffe à reflux 2 heures, refroidit à - 70°C et introduit du CO₂ gazeux durant une heure. On laisse remonter la température à 20°C, jette dans l'eau, acidifie à pH = 1 avec de l'acide chlorhydrique concentré et extrait avec de l'éther éthylique. On décante la phase organique, sèche sur sulfate de magnésium et évapore. Après recristallisation dans l'acétate d'éthyle, on obtient 37g de produit attendu (Rdt 86%) de point de fusion : 238-239°C.

### (b) Chlorure de 3-(1-adamantyl)-4-méthoxybenzoyle.

Dans un ballon, on introduit 200ml de chlorure de thionyle et ajoute par petites quantités 35g (122 mmoles) de l'acide obtenu ci-dessus. On chauffe à reflux jusqu'à cessation du dégagement gazeux. On évapore à sec, reprend par 100 ml de benzène anhydre et évapore à nouveau à sec. On obtient 37g de produit attendu (Rdt 100%) de point de fusion : 153-154°C.

### (c) 4-[3-(1-adamantyl)-4-méthoxybenzamido]benzoate de méthyle.

Dans un ballon, on introduit 2,5g (17 mmoles) de p-amino benzoate de méthyle, 50ml de tétrahydrofuranne et 2,6ml (18,5 mmoles) de triéthylamine. On ajoute goutte à goutte 5,64g (18,5 mmoles) de chlorure de l'acide 3-adamantyl-4-méthoxy benzoïque dans 50ml de tétrahydrofuranne et agite à température ambiante deux heures. On jette dans l'eau, extrait au chlorure de méthylène, décante la phase organique, sèche sur sulfate de magnésium et évapore. Après recristallisation dans un mélange éther isopropylique-acétate d'éthyle (50-50), on obtient 7,1g de produit attendu (Rdt 92%) de point de fusion : 179-180°C.

### EXEMPLE 2

### Acide 4-[3-(1-adamantyl)-4-méthoxybenzamido]benzoïque

Dans un ballon, on introduit 6g (14 mmoles) de l'ester obtenu à l'exemple 1 et 200ml de soude méthanolique 2M. On chauffe à reflux quatre heures. On évapore à sec, reprend par de l'eau, acidifie avec de l'acide chlorhydrique à pH=1, extrait à l'éther. On décante la phase organique, sèche sur sulfate de magnésium et évapore. Après recristallisation dans l'acétate d'éthyle, on obtient 4g de l'acide attendu (Rdt 69%) de point de fusion : 286-287°C.

### EXEMPLE 3

### 4-[3-(1-adamantyl)-4-hydroxybenzamido]benzoate de méthyle

### (a) Acide 3-(1-adamantyl)-4-tert-butyldiméthylsilyloxybenzoïque.

Dans un ballon, on introduit 1,18g (48,8 mmoles) de magnésium et 20ml de THF. On ajoute goutte à goutte 13,7g (32,5 mmoles) d'éther tertbutyl diméthylsilique du 2-(1-adamantyl)-4-bromophénol (décrit dans la demande européenne n°86/400785.1) et chauffe à reflux pendant 2 heures. On refroidit à -70°C et fait passer un courant de CO₂ pendant 1 heure. On laisse remonter la température à 20°C, jette le milieu réactionnel dans l'eau, acidifie à pH=1 (avec HCl concentré), et extrait à l'éther éthylique. On décante la phase organique, lave à l'eau, sèche sur sulfate de magnésium et évapore les solvants. Le résidu est trituré dans 200ml d'éther isopropylique à reflux. Après refroidissement, on filtre le précipité. On obtient ainsi 8,20g (65%) d'acide 3-(1-adamantyl)-4-tert-butyldiméthylsilyloxybenzoïque qui fond à 245-246°C.

### (b) Chlorure de l'acide 3-(1-adamantyl)-4-tert-butyldiméthyl silyloxybenzoïque.

6,45g (16,7 mmole) de l'acide obtenu en 3(a) sont mis en suspension dans 100ml de CH₂Cl₂. On ajoute 3,3ml (16,7 mmole) de dicyclohexylamine et agite 1 heure à 20°C. On ajoute alors 1,35 ml (18,4 mmole) de chlorure de thionyle. On agite 2 heures à température ambiante, évapore à sec, reprend par 300ml d'éther, filtre le sel formé et évapore la phase éthérée. On obtient ainsi 6,9g (100%) de chlorure de l'acide 3-(1-adamantyl)-4-tert-butyldiméthylsilyloxybenzoïque, sous la forme d'un solide qui est utilisé tel quel pour la suite de la synthèse.

### (c) 4-[3-(1-adamantyl)-4-tert-butyl-diméthylsilyloxybenzamido] benzoate de méthyle.

Dans un ballon, on introduit 2,10g (13,9 mmoles) de p-aminobenzoate de méthyle, 2,10ml (15,3 mmoles) de triéthylamine et 50ml de THF. On ajoute goutte à goutte 6,20g (15,3 mmoles) de chlorure de 3-(1-adamantyl)-4-méthoxybenzoyle et agite à température ambiante pendant 4 heures.

On jette le milieu réactionnel dans l'eau et extrait au chlorure de méthylène. On sèche (MgSO₄) et évapore les solvants. Le solide obtenu est recristallisé dans un mélange d'éther diisopropylique et d'acétate d'éthyle (10-1) pour donner 6,5g (91%) de l'ester attendu, qui fond à 183-184°C.

### (d) 4-[3-(1-adamantyl)-4-hydroxybenzamido]benzoate de méthyle.

Dans un ballon, on introduit 6,40g (12,3 mmoles) d'ester obtenu en 3 (c) et 75ml de THF. On ajoute goutte à goutte 13,5 ml (13,5 mmoles) de fluorure de tétrabutylammonium (1M dans le THF). On agite à température ambiante pendant 2 heures, puis jette le milieu réactionnel dans l'eau, extrait au chlrorure de méthylène, décante la phase organique, sèche sur sulfate de magnésium, et évapore les solvants.

On triture le solide obtenu dans 200ml d'acétate d'éthyle à reflux, refroidit et filtre. On obtient ainsi 4,20g (84%) d'ester méthylique de l'acide 4-[3-(1-adamantyl)-4-hydroxybenzamido]benzoïque qui fond à 305-306°C.

### EXEMPLE 4

### Acide 4-[3-(1-adamantyl)-4-hydroxybenzamido]-benzoïque

Une suspension de 3,3g (8,1 mmoles) d'ester obtenu en 3 (d) dans 100 ml de soude méthanolique 2N est agitée pendant 12 heures à température ambiante. On évapore à sec, reprend par l'eau et acidifie à pH : 0 avec de l'acide chlorhydrique concentré. Le solide est filtré, lavé à l'eau et séché sous vide en présence de pentoxyde de phosphore (P₂O₅). Le solide est ensuite trituré dans 200ml d'acétate d'éthyle à reflux. Le mélange est refroidit à température ambiante puis le précipité est filtré. On obtient ainsi 2,8g (88%) d'acide 4-[3-(1-adamantyl)-4-hydroxybenzamido]benzoïque qui fond à 348-350°C.

### EXEMPLE 5

### 4-[3-(1-méthylcyclohexyl)-4-méthoxybenzamido]benzoate d'éthyle

### (a) 4-bromo-2-(1-méthylcyclohexyl)phénol.

Un mélange de méthylène cyclohexane (0,96g, 10mmoles) de p-bromophénol (1,73g, 10mmoles) et de résine acide (Dowex 50x12) (150mg) est chauffé à 80°C pendant 16 heures. Le résidu est purifié par chromatographie sur colonne de silice (éluant : CH₂Cl₂/hexane 50/50). Par évaporation des solvants on obtient 0,50g (19%) de 4-bromo-2-(1-méthylcyclohexyl)phénol sous la forme d'une huile jaunâtre.

### (b) 4-bromo-2-(1-méthylcyclohexyl)anisole.

Le 4-bromo-2-(1-méthylcyclohexyl)phénol (9,26g, 34,4 mmoles) est dissous dans 50ml de THF. On refroidit à 0°C et ajoute par petites portions de l'hydrure de sodium (80% dans l'huile, 1,14g, 37,8 mmoles). On agite trente minutes à température ambiante et ajoute goutte à goutte 5,37g (37,8 mmoles) d'iodure de méthyle. On continue l'agitation pendant 16 heures, ajoute de l'eau (300ml) et extrait par de l'éther (3x300ml). La phase organique est lavée par une solution saturée de bicarbonate de sodium, puis une solution saturée de chlorure de sodium. On sèche (MgSO₄), filtre et évapore les solvants. Le résidu est purifié par chromatographie sur colonne de silice, élué par un mélange composé de dichlorométhane et d'hexane (20/80). On obtient ainsi 9g (92%) de 4-bromo-2-(1-méthylcyclohexyl)anisole sous forme d'une huile incolore.

### (c) Acide 3-(1-méthylcyclohexyl)-4-méthoxybenzoïque.

Le composé obtenu en 5 (b) (9,0g, 31,8 mmoles) est dissous dans 50ml de THF sec. La solution obtenue est ajoutée goutte à goutte sur du magnésium (850mg, 35 mmoles) et un cristal d'iode. On chauffe à reflux après addition des 5 premiers millilitres de solution. Le reflux est maintenu pendant 15mn après que l'addition soit terminée. On refroidit alors à - 40°C et fait passer un courant de CO₂ pendant une heure, puis jette le mélange dans le l'acide chlorhydrique 6N, extrait par de l'éther (3x300ml). La phase organique est lavée à l'eau jusqu'à neutralité, séchée (MgSO₄), et évaporée. Le résidu obtenu est trituré dans l'hexane, filtré et séché. On obtient ainsi 6,50g (82%) d'acide 3-(1-méthylcyclohexyl)-4 -méthoxybenzoïque, qui fond à 199°C.

### (d) Chlorure de 3-(1-méthylcyclohexyl)-4-méthoxybenzoyle.

Dans un ballon, on introduit 4,96g (20mmoles) d'acide 3-(1-méthyl cyclohexyl)-4-méthoxybenzoïque, 75ml de dichlorométhane, et on ajoute 4ml (20mmoles) de dicyclohexylamine. On agite pendant 1 heure. A la solution ainsi obtenue, on ajoute 1,45ml (20mmoles) de chlorure de thionyle (SOCl₂) et agite 2 heures à température ambiante. On évapore à sec, reprend par 200ml d'éther, filtre le chlorure de dicyclohexylammonium puis évapore le solvant. On obtient ainsi 5,30g (100%) de chlorure de 3-(1-méthylcyclohexyl)-4-méthoxybenzoyle brut qui est utilisé tel quel pour la suite de la synthèse.

### (e) 4-[3-(1-méthylcyclohexyl)-4-méthoxybenzamido]benzoate d'éthyle.

Dans un ballon, on introduit 3,3g (20mmoles) de p-aminobenzoate d'éthyle, 3,1ml (20mmoles) de triéthylamine et 75ml de THF. On ajoute goutte à goutte 5,3g (20mmoles) du chlorure d'acide obtenu en 5 (d) dissous dans 50ml de THF et agite à température ambiante pendant 2 heures. On jette le milieu réactionnel dans l'eau, extrait au chlorure de méthylène, décante la phase organique, sèche (MgSO₄) et évapore les solvants. On obtient ainsi 6,30g (80%) de 4-[3-1-méthylcyclohexyl)-4-méthoxybenzamido]benzoate d'éthyle, sous la fome d'une huile.

### EXEMPLE 6

### Acide 4-[3-(1-méthylcyclohexyl)-4-méthoxybenzamido]benzoïque

Dans un ballon, on introduit 5,20g (13,1 mmoles) de l'ester obtenu en 5 (e) et 150ml de soude méthanolique 2N. On agite à température ambiante 24 heures, évapore à sec, reprend par l'eau, acidifie à pH 0 avec de l'acide chlorhydrique concentré, extrait à l'éther, sèche (MgSO₄) et évapore. Le résidu est recristallisé dans un mélange d'éther isopropylique et d'acétate d'éthyle (8/2). On obtient ainsi 3,9g (82%) d'acide 4-[3-(1-méthylcyclohexyl)-4-méthoxybenzamido]benzoïque, qui fond à 230-231°C.

### EXEMPLE 7

### 4-[3-(1-adamantyl)-4-décyloxybenzamido]benzoate de méthyle

2,00g (5mmoles) d'ester obtenu en 3 (d) sont mis en solution dans 70ml de diméthylformamide (DMF) et ajoutés goutte à goutte à une suspension d'hydrure de sodium (80% dans l'huile, 150mg, 5mmoles) dans 20ml de DMF. On agite à température ambiante jusqu'à ce que le dégagement gazeux soit terminé, puis ajoute 1,1ml (5mmoles) de 1-iododécane, et agite 4 heures à température ambiante. On jette le milieu réactionnel dans l'eau, extrait à l'éther, décante la phase organique, lave à l'eau, sèche (MgSO₄) et évapore les solvants. Le résidu est purifié par chromatographie sur colonne de silice (éluant : CH₂Cl₂). On obtient ainsi 2,5g (92%) de 4-[3-(1-adamantyl)-4-décyloxybenzamido]benzoate de méthyle, qui fond à 106-107°C.

### EXEMPLE 8

### Acide 4-[3-(1-adamantyl)-4-décyloxybenzamido]benzoïque

De manière analogue à l'exemple 4, 2,00g (3,67 mmoles) d'ester obtenu en 7, traités pendant 48 heures par 100ml de soude méthanolique 2N donnent 1,8g (95%) d'acide 4-[3-(1-adamantyl)-4-décyloxybenzamido]benzoïque qui fond à 247-248°C.

### EXEMPLE 9

### 4-[3-(1-adamantyl)-4-hexyloxybenzamido]benzoate de méthyle

De manière analogue à l'exemple 7, à partir de 2,50g (6,2 mmoles) d'ester obtenu en 3 (d) traité par 187mg (6,2 mmoles) d'hydrure de sodium (80% dans l'huile) et 0,9ml (6,2mmoles de 1-iodohexane), on obtient 2,9g (96%) de 4-[3-(1-adamantyl)-4-hexyloxybenzamido]benzoate de méthyle, qui fond à 154-155°C.

### EXEMPLE 10

### Acide 4-[3-(1-adamantyl)-4-hexyloxybenzamido]benzoïque

De manière analogue à l'exemple 8, à partir de 2,27g (4,6 mmoles) d'ester obtenu en 9, on obtient 2,10g (96%) d'acide 4-[3-(1-adamantyl)-4-hexyloxybenzamido]benzoïque, qui fond à 256-257°C.

### EXEMPLE 11

### 4-[3-(1,1-diméthyldécyl)-4-méthoxybenzamido]benzoate de méthyle

### (a) 4-bromo-2-(1,1-diméthyldécyl)phénol.

Un mélange de p-bromophénol (25,85g, 149 mmoles) et de 2-méthylundec-1-ène (25,15 g, 149 mmoles) est agité à 110°C pendant 48 heures en présence de résine acide (Dowex 50x12, 3g). Le mélange obtenu est purifié par chromatographie sur colonne de silice, (éluant : dichlorométhane, hexane 50/50). On obtient ainsi 25,04g (49%) de 4-bromo-2 -(1,1-diméthyldécyl)phénol sous forme d'une huile jaune clair.

### (b) 4-bromo-2-(1,1-diméthyldécyl)anisole.

A une solution de phénol obtenu en 11 (a) (24,88g, 72,9 mmoles) dans le THF (200ml), on ajoute par petites fractions 2,19g (72,9 mmoles) d'hydrure de sodium (80% dans l'huile). Une fois l'addition terminée, on agite pendant 1 heure à température ambiante, puis ajoute goutte à goutte de l'iodure de méthyle (10,35g, 72,9 mmoles). Le milieu réactionnel est agité pendant 2 heures à température ambiante, le solvant est évaporé puis on ajoute de l'eau (300ml) et extrait à l'éther (3x200ml). La phase organique est lavée avec une solution saturée de chlorure de sodium, séchée (MgSO₄) et les solvants évaporés. On obtient ainsi 22,2g (86%) de 4-bromo-2-(1,1-diméthyldécyl)anisole sous forme d'une huile jaune.

### (c) Acide 3-(1,1-diméthyldécyl)-4-méthoxybenzoïque.

Le 4-bromo-2-(1,1-diméthyldécyl)anisole (15,72g, 44,2 mmoles) est dissous dans le THF (50ml). On ajoute cette solution goutte à goutte sur du magnésium (1,18g, 48,7 mmoles) et un cristal d'iode, tout en maintenant au reflux par chauffage. Une fois l'addition terminée, on maintient au reflux pendant 30mn puis refroidit à -40°C. On ajoute alors 30ml de THF et fait passer un courant de CO₂ pendant 2 heures. Le mélange réactionnel est ensuite versé dans une solution d'acide chlorhydrique (4N, 300ml), et le produit est extrait par de l'éther (3x300ml). La phase organique est lavée à l'eau jusqu'à neutralité, séchée (MgSO₄), puis les solvants sont évaporés. Le résidu est trituré dans l'isooctane pour donner 7,25g (51%) d'acide 3-(1,1-diméthyldécyl)-4-méthoxybenzoïque, qui fond à 112°C.

### (d) Chlorure de 3-(1,1-diméthyldécyl)-4-méthoxybenzoyle.

L'acide obtenu en 11 (c) (7,18g, 22,4 mmoles) est mis en suspension dans 200ml de dichlorométhane. On ajoute goutte à goutte de la dicyclohexylamine (4,06g, 22,4 mmoles) puis refroidit le mélange à 0°C. On ajoute alors du chlorure de thionyle (2,66g, 22,4 mmoles) et agite pendant 16 heures à température ambiante. Le précipité formé est filtré, le solvant évaporé. On obtient ainsi quantitativement le chlorure de 3-(1,1-diméthyldécyl)-4-méthoxybenzoyle brut sous forme d'un solide blanc qui est utilisé tel quel pour la suite de la synthèse.

### (e) 4-[3-(1,1-diméthyldécyl)-4-méthoxybenzamido]benzoate de méthyle.

La totalité du chlorure d'acide obtenu en 11 (d) est dissous dans 50ml de THF. La solution ainsi obtenue est ajoutée à une solution de p-aminobenzoate de méthyle (3,39g, 22,4 mmoles) et de triéthylamine (2,27g, 22,4 mmoles) dans le THF (100ml). On agite pendant 1 heure à température ambiante, puis le précipité est filtré, le solvant évaporé et le produit purifié par chromatographie sur colonne (éluant : dichlorométhane). Les solvants sont évaporés et le solide obtenu est trituré dans l'hexane, filtré et séché. On obtient ainsi 7,72g (76%) de 4-[3-(1,1-diméthyldécyl) -4-méthoxybenzamido]benzoate de méthyle qui fond à 120°C.

### EXEMPLE 12

### Acide 4- 3-(1,1-diméthyldécyl)-4-méthoxybenzamido benzoïque

L'ester obtenu en 11 (e) (2,5g, 5,51mmoles) est mélangé avec 110ml de méthanol. On ajoute 11ml de soude 5N et agite le milieu réactionnel pendant trois jours. Le méthanol est évaporé, on ajoute de l'acide chlorhydrique 4N (200ml) et extrait le produit avec du dichlorométhane (3x300ml). La phase organique est lavée avec une solution saturée de bicarbonate de sodium, puis de chlorure de sodium. On sèche (MgSO₄) puis évapore le solvant. Le solide obtenu est trituré dans l'hexane, filtré puis séché. On obtient ainsi 1,57g (65%) d'acide 4-[3-(1,1-diméthyldécyl)-4-méthoxybenzamido]benzoïque, qui fond à 177°C.

### EXEMPLE 13

### 4-(3-tert-butyl-4-méthoxybenzamido)benzoate de méthyle

Le chlorure de l'acide 3-(tert-butyl)-4-méthoxy benzoïque brut préparé à partir de 10,41g (50mmoles), d'acide 3-(tert-butyl) -4-méthoxybenzoïque décrit dans la demande de brevet français n°85.13747 (2.570.377) , est dissous dans du THF (60ml). La solution est ajoutée goutte à goutte sur un mélange de 4-aminobenzoate de méthyle (7,14g ; 47,2 mmoles) et de triéthylamine (4,78g, 47,2 mmoles), en solution dans le THF (50ml). On agite 3 heures à température ambiante, filtre le précipité formé, puis évapore les solvants. On ajoute de l'eau (300ml) et extrait le produit par de l'éther (3x200ml).

La phase organique est lavée avec une solution saturée de bicarbonate de sodium, puis de chlorure de sodium. On sèche (MgSO₄), filtre et évapore les solvants. Le solide obtenu est recristallisé dans de l'hexane contenant environ 5% de méthanol. On obtient ainsi 14,02g (87%) de 4-(3-tert-butyl-4-méthoxybenzamido)benzoate de méthyle.

### EXEMPLE 14

### Acide 4-(3-tert-butyl-4-méthoxybenzamido)benzoïque

De manière analogue à l'exemple 4, à partir de 5g (14,65 mmoles) d'ester obtenu à l'exemple 13, on obtient 4,27g (89%) d'acide 4-(3-tert-butyl-4-méthoxybenzamido)benzoïque qui fond à 250°C.

### EXEMPLE 15

### N-[4-[3-(1-adamantyl)-4-méthoxybenzamido]-benzoyl]pyrrolidine

1,7g (9mmoles) de N- p-aminobenzoyl pyrrolidine, et 1g (10mmoles) de triéthylamine sont dissous dans 30ml de dichlorométhane. Sous agitation, on ajoute 2,8g (9mmoles) de chlorure de 3-(1-adamantyl)-4-méthoxybenzoyle dissout dans 60ml de dichlorométhane. On agite pendant 16 heures, ajoute de l'eau et extrait au dichlorométhane. On lave à l'eau, extrait au chlorure de méthylène, sèche (MgSO₄) puis évapore les solvants. Le résidu (mousse jaune) est cristallisé dans l'acétate d'éthyle pour donner 3,0g (73%) de N-[4-[3-(1-adamantyl)-4-méthoxybenzamido]-benzoyl] pyrrolidine qui fond à 239-242°C.

### EXEMPLE 16

### N-[4-[3(1-adamantyl)-4-méthoxybenzamido]-benzoyl]pipéridine

De manière analogue à l'exemple 15, à partir de 0,7g (3,6 mmoles) de N-[4-aminobenzoyl]pipéridine, on obtient 1,0 g (63%) de N-[4-[3-(1-adamantyl)-4-méthoxybenzamido]-benzoyl]pipéridine, qui fond à 219-222°C.

### EXEMPLE 17

### Morpholide de l'acide 4-[3-(1-adamantyl)-4-méthoxybenzamido] benzoïque

De manière analogue à l'exemple 15, à partir de 3,0g (15mmoles) de N-[4-aminobenzoyl]morpholine, on obtient 5,6g (81%) de morpholide de l'acide 4-[3-(1-adamantyl)-4-méthoxybenzamido]benzoïque, qui fond à 238-241°C.

### EXEMPLE 18

### Amide tert-butylique de l'acide 4-[3-(1-adamantyl) -4-méthoxybenzamido]benzoïque

A partir de 1,0g (5mmoles) de N-tert-butyl- 4-aminobenzamide , on obtient 1,5g (63%) d'amide tert-butylique de l'acide 4-[3-(1-adamantyl) -4-méthoxybenzamido]benzoïque, qui fond à 270-273°C.

### EXEMPLE 19

### Amide éthylique de l'acide 4-[3-(1-adamantyl)-4-méthoxybenzamido] benzoïque

### a) Chlorure de l'acide 4-[3-(1-adamantyl)-4-méthoxybenzamido] benzoïque

2,0g (5mmoles) d'acide 4-[3-(1-adamantyl)-4-méthoxybenzamido] benzoïque sont dissous dans 60ml de THF. On ajoute goutte à goutte 1,1g (6mmoles) de dicyclohéxylamine. Un précipité blanc se forme immédiatement. On refroidit alors à 0°C et ajoute goutte à goutte 0,7g (6mmoles) de chlorure de thionyle. On agite pendant 3 heures à température ambiante, filtre le solide formé puis évapore le filtrat. Le résidu obtenu est utilisé tel quel pour la suite de la synthèse.

### b) Amide éthylique de l'acide 4-[3-(1-adamantyl)-4-méthoyxbenzamido]benzoïque

Le chlorure d'acide brut obtenu est dissous dans 80ml de THF puis ajouté goutte à goutte à une solution d'éthylamine (0,5g , 11mmoles) dans du THF sec (20ml). On agite pendant 16 heures à température ambiante, filtre et évapore le filtrat. Le résidu rougeâtre ainsi obtenu est recristallisé dans l'éthanol pour donner 0,5g (24%) d'amide éthylique de l'acide 4-[3-(1-adamantyl)-4-méthoxybenzamido]benzoïque qui fond à 274-277°C.

### EXEMPLE 20

### Anilide de l'acide 4-[3-(1-adamantyl)-4-méthoxybenzamido] benzoïque

Ce composé est obtenu selon le même mode opératoire que celui décrit à l'exemple 19. On obtient 0,7g (30%) du composé attendu qui fond à 265-268°C.

### EXEMPLE 21

### Amide benzylique de l'acide 4-[3-(1-adamantyl)-4-méthoxybenzamido]benzoïque

Ce composé est obtenu selon le même mode opératoire que celui décrit à l'exemple 19. On obtient 0,2g (9%) du produit attendu qui fond à 279-280°C.

### EXEMPLE 22

### 4-[3-(1-adamantyl)-4-méthyoxybenzamido]benzoate de 2-hydroxyéthyle

Le chlorure d'acide brut obtenu à l'exemple 19 (a) à partir de 2g d'acide 4-[3-(1-adamantyl)-4-méthoxybenzamido]benzoïque est dissous dans une solution d'éthylène glycol (1,4g , 22mmoles) et de pyridine (0,8g , 10mmoles) dans du THF sec (20ml). On agite 16 heures à température ambiante. Le mélange réactionnel est filtré, le filtrat évaporé à sec pour donner un résidu jaunâtre que l'on purifie par chromatographie sur colonne en utilisant comme éluant un mélange de dichlorométhane et d'acétate d'éthyle (1/1). On évapore les solvants et obtient ainsi 1,2g (55%) d'ester attendu qui fond à 201-203°C.

### EXEMPLE 23

### N-(4-acétylphényl)-3-(1-adamantyl)-4-méthoxybenzamide

Une solution de 5,7g de chlorure de 3-(1-adamantyl) -4-méthoxybenzoyle obtenu à l'exemple 1 (b) dans du dichlorométhane (60ml) est ajouté goutte à goutte à un mélange de 4-aminoacétophénone (2,6g, 19 mmoles) et de triéthylamine (2,1g, 21mmoles) dans du dichlorométhane (30ml). Le mélange est agité pendant 16 heures puis jeté dans de l'eau et extrait avec du dichlorométhane. La phase organique est recueillie, lavée à l'eau, séchée sur sulfate de magnésium puis évaporée. Le résidu ainsi obtenu est recristallisé dans l'acétate d'éthyle et on obtient ainsi 3,0g (39%) de N-(4-acétylphényl)-3-(1-adamantyl)-4-méthoxybenzamide sous la forme de cristaux blancs de point de fusion 200-201°C.

### EXEMPLE 24

### M-[4-(1-hydroxyéthyl)phényl]-3-(1-adamantyl)-4-méthoxybenzamide

L'amide obtenu à l'exemple 23 (0,9g, 2mmoles) est dissous dans du méthanol (25ml) et traité par 0,12g (3mmoles) de borohydrure de sodium. Le mélange est agité à température ambiante pendant 2 jours, jeté dans de l'eau et extrait à l'éther. Les extraits sont séchés sur sulfate de magnésium, puis le solvant évaporé. Le résidu ainsi obtenu est recristallisé dans l'acétate d'éthyle pour donner le N-[4-(1-hydroxyéthyl)phényl]-3-(1-adamantyl)-4-méthoxybenzamide (0,5g , 66%) de point de fusion : 207-209°C.

### EXEMPLE 25

### Amide 2-hydroxyéthylique de l'acide 4-[3-(1-adamantyl)-4 - méthoxybenzamido]benzoïque

De manière analogue à l'exemple 19, on obtient l'amide 2-hydroxyéthylique de l'acide 4-[3-(1-adamantyl)-4-méthoxybenzamido] benzoïque : (1,1g, 50%) qui fond à 265-268°C (cristallisé dans un mélange éthanol-éther).

### EXEMPLE 26

### 2-hydroxy-4[3-(1-adamantyl)-4-méthoxybenzamido]benzoate de méthyle

### a) 4-amino-2-tert-butyldiméthylsilyloxy benzoate de méthyle.

2,0 g (12mmoles) de 4-amino-2-hydroxybenzoate de méthyle sont dissous dans 30ml de diméthylformamide (DMF) contenant 2,8g (28mmoles de triéthylamine, et 70mg (0,6 mmoles) de 4-N,N-diméthylaminopyridine. On ajoute une solution de chlorure de tert-butyldiméthylsilyle (4,2g, 28mmoles) dans 40ml de DMF, goutte à goutte. On agite pendant 2 jours à température ambiante, puis chauffe à 100°C pendant 8 heures. Le DMF est évaporé sous vide, on ajoute de l'eau et extrait à l'éther. La phase organique est recueillie, séchée puis le solvant évaporé. On obtient ainsi le 4-amino-2-tert-butyldiméthylsilyloxybenzoate de méthyle, brut, qui est utilisé tel quel pour la suite de la synthèse.

### b) 2-tert-butyldiméthylsilyloxy-4-[3-(1-adamantyl)-4-méthoxybenzamido]benzoate de méthyle.

Le 4-amino-2-tert-butyldiméthylsilyloxy benzoate de méthyle brut (3,0g, 10mmoles) est dissous dans 20ml de T.H.F. sec contenant 1,1g (10mmoles) de triéthylamine. On ajoute goutte à goutte une solution de chlorure de 4-méthoxy-3-(1-adamantyl)bensoyle dans 80ml de T.H.F. sec et agite le milieu réactionnel pendant 16 heures à 20°C. On évapore à sec, reprend par 100ml de dichlorométhane, lave à l'eau, sèche (Mg SO₄) et évapore à sec. Le résidu ainsi obtenu est recristallisé dans un mélange éthanol/éther éthylique. On obtient ainsi 2,7g (48%) de 2-tert-butyldiméthylsilyloxy-4-[3-(1-adamantyl)-4-méthoxybenzamido] benzoate de méthyle qui fond à 225-227°C.

### c) 2-hydroxy-4-[3-(1-adamantyl)-4-méthoxybenzamido]benzoate de méthyle.

2,7g (5mmoles) d'ester obtenu (b) ci-dessus, sont dissous dans 80ml de T.H.F.. On ajoute une solution 1M de fluorure de tétrabutylammonium dans le T.H.F. (6ml). On agite 16 heures à 20°C (on observe la formation d'un précipité blanc). On évapore à sec, ajoute de l'eau et extrait à l'éther (3x100ml). On sèche (Mg SO₄) et évapore à sec (2,0g , 95%) ; le résidu est cristallisé par addition d'une petite quantité d'éther. On obtient ainsi le 2-hydroxy-4-[3-(1-adamantyl)-4-méthoxybenzamido]benzoate de méthyle (1,6g , 76%) qui fond à 207-209°C.

### EXEMPLES DE FORMULATION

### A. VOIE ORALE

### (a) comprimé de 0,2g

| | |
|---|---|
| - 4-[3-(1-adamantyl)-4-méthoxybenzamido] benzoate de méthyle | 0,001g |
| - Amidon | 0,114g |
| - Phosphate bicalcique | 0,020g |
| - Silice | 0,020g |
| - Lactose | 0,030g |
| - Talc | 0,010g |
| - Stéarate de magnésium | 0,005g |

### (b) Suspension buvable en ampoules de 5ml

| | |
|---|---|
| - Acide 4-[3-(1-adamantyl)-4-méthoxy benzamido]benzoïque | 0,001g |
| - Glycérine | 0,500g |
| - Sorbitol à 70% | 0,500g |
| - Saccharinate de sodium | 0,010g |
| - Parahydroxybenzoate de méthyle | 0,040g |
| - Arôme qs | |
| - Eau purifiée q.s.p | 5 ml |

### B. VOIE TOPIQUE

### (a) Onguent

| | |
|---|---|
| - Acide 4-[3-(1-adamantyl)-4-méthoxybenzamido] benzoïque | 0,020g |
| - Myristate d'isopropyle | 81,700g |
| - Huile de vaseline fluide | 9,100g |
| - Silice vendue par la Société DEGUSSA sous le nom de "Aérosil 200" | 9,180g |

### (b) Onguent anhydre hydrophobe

| | |
|---|---|
| - Acide 4-[3-(1,1-diméthyldécyl)-4-méthoxybenzamido]benzoïque | 0,10g |
| - Vaseline blanche | 49,95g |

Cet onguent est obtenu par mélange de la vaseline et du "Miglyol 812" à 70°C. On introduit ensuite le composé actif en le dispersant très soigneusement avec un bain à ultrasons en chauffant à 40°C/50°C. On refroidit ensuite sous agitation.

Dans cet exemple, le composé actif (0,10g) peut être remplacé par 0,5g d'acide 4-[3-(1-adamantyl)-4-hexyloxybenzamido]benzoïque.

### (c) Lotion

| | |
|---|---|
| - Acide 4-[3-(1-méthylcyclohexyl) -4-méthoxybenzamido]benzoïque | 0,01g |
| - Ethanol absolu | 30,00g |
| - Polyéthylène glycol (400) | 69,99g |

Cette lotion est obtenue par mélange du polyéthylène glycol (400) et de l'éthanol puis on introduit le composé actif et on le solubilise dans un bain à ultrasons.

### (d) Emulsion anionique huile-dans-l'eau

| | |
|---|---|
| - Laurylsulfate de sodium | 0,784g |
| - 1,2-propanediol | 1,570g |
| - Vaseline blanche | 19,502g |
| - Alcool cétylique | 19,504g |
| - Parahydroxybenzoate de méthyle | 0,076g |
| - Parahydroxybenzoate de propyle | 0,074g |
| - Morpholide de l'acide 4-[3-(1-adamantyl) -4-méthoxybenzamido]benzoïque | 0,500g |
| - Eau stérile q.s.p. | 100,00g |

Cette émulsion est obtenue par préparation des mélanges A et B suivants :

### MELANGE A

- Laurylsulfate de sodium
- 1,2 propanediol
- Parahydroxybenzoate de méthyle
- Eau stérile

Après avoir dissous le parahydroxybenzoate de méthyle sous agitation à ultrasons, on porte le mélange à 75°C.

### MELANGE B

- Vaseline blanche
- Alcool cétylique
- Parahydroxybenzoate de propyle

Après avoir dissous le parahydroxybenzoate de propyle sous agitation à ultrasons, on porte également le mélange à 75°C.

On procède alors à la formation de l'émulsion en versant le mélange A dans le mélange B. Après refroidissement à température ambiante, on incorpore le composé actif et agite soigneusement pour bien homogénéiser. Avant de conditionner l'émulsion, on la passe au tricylindre.

### C - COMPOSITIONS COSMETIQUES

### (a) Crème fluide non grasse

| | |
|---|---|
| - 4-[3-(1-adamantyl)-4-hexyloxybenzamido] benzoate de méthyle | 1,00g |
| - Palmito stéarate d'éthylène glycol et de polyoxyéthylène glycol | 20,00g |
| - Glycérides saturés de C₁₀-C₁₈ polyoxyéthylénés glycolisés | 3,00g |
| - Huile de vaseline fluide | 3,00g |
| - Conservateurs | 0,05g |
| - Eau q.s.p. | 100,00g |

Cette crème est obtenue en portant à 70°C un mélange du Palmito stéarate d'éthylène glycol et de polyoxyéthylène glycol, des glycérides saturés C₁₀-C₁₈ polyoxyéthylénés glycolisés et de l'huile de vaseline. On introduit alors le composé actif et l'on disperse soigneusement. On verse alors à la phase grasse sous agitation l'eau et les conservateurs portés également à 70°C. On continue l'agitation jusqu'à retour à la température ambiante et l'on obtient une émulsion.

Dans cet exemple, le composé actif peut être remplacé par la même quantité du morpholide de l'acide 4-[3-(1-adamantyl)-4 méthoxybenzamido] benzoïque.

### (b) Crème consistante peu grasse

| | |
|---|---|
| - Acide 4-(3-tert-butyl-4-méthoxybenzamido) benzoïque | 1,00g |
| - Mélange mono et diglycérides d'acides palmitique et stéarique | 15,00g |
| - Monostéarate de Sorbitan | 4,00g |
| - Monostéarate de Sorbitan polyoxyéthyléné à 20moles d'oxyde d'éthylène | 1,20g |
| - Huile de vaseline fluide | 10,00g |
| - Conservateurs | 0,04g |
| - Eau q.s.p. | 100,00g |

Cette crème est obtenue selon le même mode opératoire que celui décrit ci-dessus.

Dans cet exemple, le composé actif peut être remplacé par la même quantité de l'amide éthylique de l'acide 4-[3-(1-adamantyl)-4-méthoxybenzamido]benzoïque.

### (c) Huile de protection solaire

| | |
|---|---|
| - 4-[3-(1-adamantyl)-4 décyloxybenzamido] benzoate de méthyle | 0,50g |
| - 2-octyl dodécanol | 42,00g |
| - Triglycérides des acides caprique et caprylique | 40,00g |
| - Mélange d'esters d'acides caprique, caprylique et d'alcools gras saturés en C₁₂-C₁₈ | 17,50g |

### (d) Lotion alcoolique pour le cuir chevelu

| | |
|---|---|
| - Acide 4-(3-tert-butyl-4-méthoxybenzamido) benzoïque | 0,80g |
| - Ethanol 95% | 83,00g |
| - Eau q.s.p. | 100,00g |

### (e) Shampooing en 2 phases à mélanger extemporanément

### (i) Phase traitante

| | |
|---|---|
| - 4-[3-(1-adamantyl)-4-décyloxybenzamido] benzoate de méthyle | 0,50g |
| - 2-octyl dodécanol | 50,00g |
| - Triglycérides des acides caprique et caprylique | 49,50g |

### (ii) Phase lavante

| | |
|---|---|
| - Lauryl éther sulfate de sodium | 50,00g |
| - Cocoate de glycérol polyoxyéthyléné à 7 moles d'oxyde d'éthylène | 5,00g |
| - Conservateurs | 0,05g |
| - Eau q.s.p. | 100,00g |

Au moment de l'emploi, on mélange 10g de la Phase traitante à 90g de la phase lavante.

## Revendications

1. Composés benzamido aromatiques, caractérisés par le fait qu'ils répondent à la formule générale suivante : 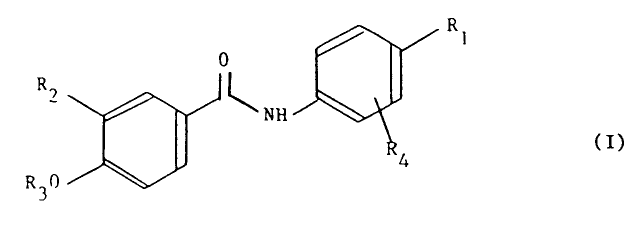 dans laquelle :
R₁ représente - CH₂OH, -CHOHCH₃ ou -COR₅,
R₅ représentant un atome d'hydrogène, un radical alkyle inférieur, le radical -OR₆ 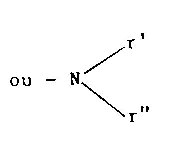 R₆ représentant un atome d'hydrogène, un radical alkyle inférieur ou un radical mono ou polyhydroxyalkyle, r' et r'' représentant un atome d'hydrogène, un radical alkyle inférieur, un radical mono ou polyhydroxyalkyle, un radical aryle ou benzyle éventuellement substitué(s), un reste d'aminoacide ou de sucre aminé ou pris ensemble forment un hétérocycle,
R₂ représente un radical alkyle α,α'disubstitué ayant de 4 à 12 atomes de carbone ou un radical cycloalkyle, mono ou polycyclique ayant de 5 à 12 atomes de carbone dant le carbone de liaison est quaternaire,
R₃ représente un atome d'hydrogène ou un radical alkyle avant de 1 à 10 atomes de carbone, et
R₄ représente un atome d'hydrogène, un radical alkyle inférieur ou un radical hydroxyle, à l'exclusion du composé de formule (I) dans laquelle R₁ représente le radical -CO₂CH₃, R₂ représente le radical 1-adamantyle, R₃ représente le radical décyle et R₄ représente le radical hydroxyle, et les sels desdits dérivés benzamido aromatiques de formule (I) lorsque R₆ représente un atome d'hydrogène.

2. Composés selon la revendication 1, caractérisés par le fait que lorsque les composés de formule (I) se présentent sous forme de sels il s'agit de sels d'un métal alcalin ou alcalino-terreux ou encore de zinc ou d'une amine organique.

3. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

4. Composés selon la revendication 1, caractérisés par le fait que le radical monohydroxyalkyle est un radical 2-hydroxyéthyle, 2-hydroxypropyle ou 3-hydroxypropyle.

5. Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxypentyle et le reste du pentaérythritol.

6. Composés selon la revendication 1, caractérisés par le fait que le radical aryle est un radical phényle éventuellement substitué par un atome d'halogène, un hydroxyle ou une fonction nitro.

7. Composés selon la revendication 1 caractérisés par le fait que le radical α,α'-disubstitué ayant de 4 à 12 atomes de carbone est un radical tert-butyle, 1,1-diméthyl propyle, 1-méthyl 1-éthyl propyle, 1-méthyl 1-éthyl hexyle ou 1,1-diméthyl décyle.

8. Composés selon la revendication 1, caractérisés par le fait que le radical cycloalkyle mono ou polycyclique, ayant de 5 à 12 atomes de carbone dont le carbone de liaison est quaternaire, est un radical 1-méthylcyclohexyle ou 1-adamantyle.

9. Composés selon la revendication 1, caractérisés par le fait que le reste d'amino acide est un reste dérivant de lysine ou de glycine.

10. Composés selon la revendication 1, caractérisés par le fait que le reste d'un sucre aminé est un reste dérivant de glucosamine, de galactosamine ou de mannosamine.

11. Composés selon la revendication 1, caractérisés par le fait que les radicaux r' et r'' pris ensemble forment un hétérocycle pris dans le groupe constitué par un radical pipéridino, pipérazino, morpholino, pyrrolidino ou (2-hydroxy éthyl)-4 pipérazino.

12. Composés selon l'une quelconque des revendications précédentes caractérisés par le fait qu'ils sont pris dans le groupe constitué par :
- l'acide 4-[3-(1-adamantyl)-4-méthoxybenzamido]benzoïque,
- le 4-[3-(1-adamantyl)-4-méthoxybenzamido]benzoate de méthyle,
- le N-éthyl 4-[3-(1-adamantyl)-4-méthoxybenzamido]benzamide
- l'acide 4-[3-(1-adamantyl)-4-hydroxybenzamido]benzoïque
- le 4-[3-(1-adamantyl)-4-hydroxybenzamido]benzoate de méthyle,
- l'acide 4-[3-(1-méthylcyclohexyl)-4-méthoxybenzamido]benzoïque
- le 4-[3-(1-méthylcyclohexyl)-4-méthoxybenzamido]benzoate d'éthyle,
- l'acide 4-[3-(1-adamantyl)-4-décyloxybenzamido] benzoïque,
- le 4-[3-(1-adamantyl)-4-décyloxybenzamido]benzoate de méthyle,
- l'acide 4-[3-(1-adamantyl)-4-hexyloxybenzamido]benzoïque
- le 4-[3-(1-adamantyl)-4-hexyloxybenzamido]benzoate de méthyle,
- l'acide 4-[3-(1,1-diméthyldécyl)-4-méthoxybenzamido] benzoïque,
- le 4-[3-(1,1-diméthyldécyl)-4-méthoxybenzamido] benzoate de méthyle
- l'acide 4-(3-tert-butyl-4-méthoxybenzamido) benzoïque,
- le 4-(3-tert-butyl-4-méthoxybenzamido)benzoate de méthyle,
- la N- [4-[3-(1-adamantyl)-4-méthoxybenzamido]-benzoyl] pyrrolidine,
- la N-[4-[3-(1-adamantyl)-4-méthoxybenzamido]-benzoyl] pipéridine,
- le morpholide de l'acide 4-[3-(1-adamantyl) -4-méthoxybenzamido]benzoïque,
- l'amide tert-butylique de l'acide 4-[3-(1-adamantyl)-4-méthoxybenzamido]benzoïque,
- l'amide éthylique de l'acide 4-[3-(1-adamantyl)-4-méthoxybenzamido]benzoïque,
- L'anilide de l'acide 4-[3-(1-adamantyl)-4-méthoxybenzamido]benzoïque,
- l'amide benzylique de l'acide 4-[3-(1-adamantyl)-4-méthoxybenzamido]benzoïque,
- le 4-[3-(1-adamantyl)-4-méthoxybenzamido]benzoate de 2-hydroxyéthyle,
- le N-(4-acétylphényl)-3-(1-adamantyl)-4-méthoxy benzamide,
- le N-[4-(1-hydroxyéthyl)phényl]-3-(1-adamantyl)-4-méthoxy benzamide.
- l'amide 2-hydroxyéthylique de l'acide 4-[3-(1-adamantyl) -4-méthoxybenzamido]benzoïque,
- le 2-hydroxy-4-[3-(1-adamantyl)-4-méthoxybenzamido]benzoate de méthyle.

13. Procédé de préparation des composés selon l'une quelconque des revendications 1 à 12, caractérisé par le fait qu'il consiste à faire réagir dans un solvant organique et en présence d'une amine tertiaire une forme activée d'un acide benzoïque substitué notamment un chlorure d'acide de formule : 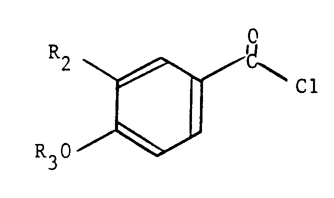 sur un composé aminé de formule : 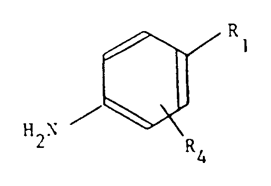 dans lesquelles :
R₁, R₂, R₃ et R₄ ont les mêmes significations que celles données à la revendication 1 la réaction étant conduite à température ambiante sous agitation.

14. Procédé selon la revendication 13 caractérisé par le fait que R₁ représente le radical -COOR₆, R₆ étant un radical alkyle inférieur et que l'ester obtenu est soumis à une hydrolyse en vue d'obtenir l'acide correspondant.

15. Procédé selon la revendication 14, caractérisé par le fait que l'acide obtenu est activé puis transformé en amide correspondant par action d'une amine de formule : 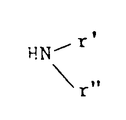 dans laquelle r' et r'' ont les mêmes significations que celles données à la revendication 1.

16. Médicament caractérisé par le fait qu'il est un composé de formule (I) selon l'une quelconque des revendications 1 à 12.

17. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 12.

18. Composition selon la revendication 17, caractérisée par le fait qu'elle se présente sous une forme appropriée pour une application topique et contient de 0,0001 à environ 5% en poids d'un composé de formule (I).

19. Utilisation d'un composé selon l'une quelconque des revendications 1 à 12 pour la préparation d'une composition pharmaceutique destinée au traitement des affections dermatologiques, rhumatismales, respiratoires ainsi qu'ophtalmologiques.

20. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 12.

21. Composition cosmétique selon la revendication 20 caractérisée par le fait qu'elle contient le composé de formule (I) à une concentration comprise entre 0,0001 et 0,1% et de préférence entre 0,001 et 0,01% en poids.

## Claims

1. Aromatic benzamido compounds, characterised in that they correspond to the following general formula: 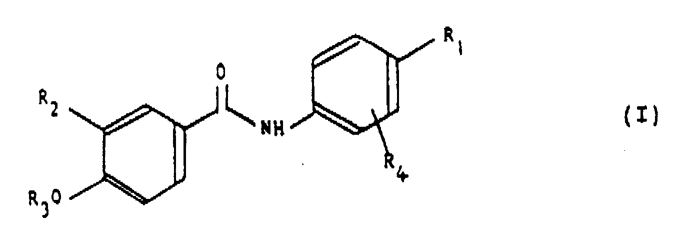 in which:
R₁ denotes -CH₂OH, -CHOHCH₃ or -COR₅,
R₅ denoting a hydrogen atom, a lower alkyl radical, the radical -OR₆ or 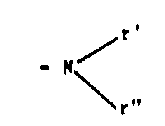 R₆ denoting a hydrogen atom, a lower alkyl radical or a mono- or polyhydroxyalkyl radical, r' and r'' denoting a hydrogen atom, a lower alkyl radical, a mono- or polyhydroxyalkyl radical, an aryl or benzyl radical, optionally substituted, an amino acid residue or an amino sugar residue or, taken together, form a heterocycle,
R₂ denotes an α, α'-disubstituted alkyl radical having from 4 to 12 carbon atoms, or a mono- or polycyclic cycloalkyl radical having from 5 to 12 carbon atoms in which the linking carbon is quaternary,
R₃ denotes a hydrogen atom or an alkyl radical having from 1 to 10 carbon atoms, and
R₄ denotes a hydrogen atom, a lower alkyl radical or a hydroxyl radical, excluding the compound of formula (I) in which R₁ denotes a -CO₂CH₃ radical R₂ denotes a 1-adamantyl radical, R₃ denotes a decyl radical and R₄ denotes a hydroxyl radical, and the salts of the said aromatic benzamido derivatives of formula (I) when R₆ denotes a hydrogen atom.

2. Compounds according to Claim 1, characterised in that, when the compounds of formula (I) take the form of salts, they are alkali metal or alkaline earth metal salts or alternatively zinc salts or salts of an organic amine.

3. Compounds according to Claim 1, characterised in that the lower alkyl radical has from 1 to 6 carbon atoms and is selected from the group consisting of methyl, ethyl, isopropyl, butyl and tert-butyl radicals.

4. Compounds according to Claim 1, characterised in that the monohydroxyalkyl radical is a 2-hydroxyethyl, 2-hydroxypropyl or 3-hydroxypropyl radical.

5. Compounds according to Claim 1, characterised in that the polyhydroxyalkyl radical contains from 3 to 6 carbon atoms and from 2 to 5 hydroxyl groups, and is selected from the group consisting of 2,3-dihydroxypropyl, 2,3,4-trihydroxybutyl and 2,3,4,5-tetrahydroxypentyl radicals and a pentaerythritol residue.

6. Compounds according to Claim 1, characterised in that the aryl radical is a phenyl radical, optionally substituted with a halogen atom, a hydroxyl or a nitro group.

7. Compounds according to Claim 1, characterised in that the α,α'-disubstituted radical having from 4 to 12 carbon atoms is a tert-butyl, 1,1-dimethylpropyl, 1-methyl-1-ethylpropyl, 1-methyl-1-ethylhexyl or 1,1,-dimethyldecyl radical.

8. Compounds according to Claim 1, characterised in that the mono- or polycyclic cycloalkyl radical having from 5 to 12 carbon atoms in which the linking carbon is quaternary is a 1-methylcyclohexyl or 1-adamantyl radical.

9. Compounds according to Claim 1, characterised in that the amino-acid residue is a residue derived from lysine or from glycine.

10. Compounds according to Claim 1, characterised in that the amino-sugar residue is a residue derived from glucosamine, from galactosamine or from mannosamine.

11. Compounds according to Claim 1, characterised in that the radicals r' and r'', taken together, form a heterocycle selected from the group consisting of a piperidino, piperazino, morpholino, pyrrolidino or 4-(2-hydroxyethyl)piperazino radical.

12. Compounds according to any one of the preceding claims, characterised in that they are selected from the group consisting of:
- 4-[3-(1-adamantyl)-4-methoxybenzamido]benzoic acid,
- methyl 4-[3-(1-adamantyl)-4-methoxybenzamido]-benzoate,
- N-ethyl-4-[3-(1-adamantyl)-4-methoxybenzamido]-benzamide,
- 4-[3-(1-adamantyl)-4-hydroxybenzamido]benzoic acid,
- methyl 4-[3-(1-adamantyl)-4-hydroxybenzamido]-benzoate,
- 4-[3-(1-methylcyclohexyl)-4-methoxybenzamido]-benzoic acid,
- ethyl 4-[3-(1-methylcyclohexyl)-4-methoxybenzamido]-benzoate,
- 4-[3-(1-adamantyl)-4-decyloxybenzamido]benzoic acid,
- methyl 4-[3-(1-adamantyl)-4-decyloxybenzamido]-benzoate,
- 4-[3-(1-adamantyl)-4-hexyloxybenzamido]benzoic acid,
- methyl 4-[3-(1-adamantyl)-4-hexyloxybenzamido]-benzoate,
- 4-[3-(1,1-dimethyldecyl)-4-methoxybenzamido]-benzoic acid,
- methyl 4-[3-(1,1-dimethyldecyl)-4-methoxybenzamido]benzoate,
- 4-(3-tert-butyl-4-methoxybenzamido)benzoic acid,
- methyl 4-(3-tert-butyl-4-methoxybenzamido)-benzoate,
- N-{4-[3-(1-adamantyl)-4-methoxybenzamido]-benzoyl}-pyrrolidone,
- N-{4-[3-(1-adamantyl)-4-methoxybenzamido]-benzoyl}-piperidine,
- 4-[3-(1-adamantyl)-4-methoxybenzamido]benzoic acid morpholide,
- 4-[3-(1-adamantyl)-4-methoxybenzamido]benzoic acid tert-butylamide,
- 4-[3-(1-adamantyl)-4-methoxybenzamido]benzoic acid ethylamide,
- 4-[3-(1-adamantyl)-4-methoxybenzamido]benzanilide,
- 4-[3-(1-adamantyl)-4-methoxybenzamido]benzoic acid benzylamide,
- 2-hydroxyethyl 4-[3-(1-adamantyl)-4-methoxybenzamido]benzoate,
- N-(4-acetylphenyl)-3-(1-adamantyl)-4-methoxybenzamide,
- N-[4-(1-hydroxyethyl)phenyl]-3-(1-adamantyl)-4-methoxybenzamide,
- 4-[3-(1-adamantyl)-4-methoxybenzamido]benzoic acid (2-hydroxyethyl)amide, and
- methyl 2-hydroxy-4-[3-(1-adamantyl)-4-methoxybenzamido]benzoate.

13. Process for preparing the compounds according to any one of Claims 1 to 12, characterised in that it consists in reacting, in an organic solvent and in the presence of a tertiary amine, an activated form of a substituted benzoic acid, in particular an acid chloride of formula: 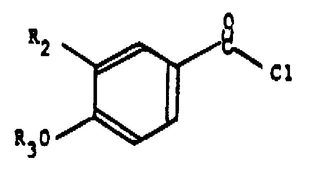 with an amino compound of formula: 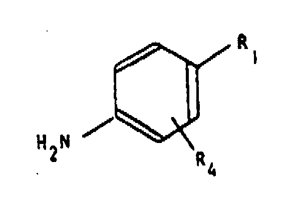 in which:
R₁, R₂, R₃ and R₄ have the same meanings as those given in Claim 1, the reaction being performed at room temperature with stirring.

14. Process according to claim 13, characterised in that R₁ denotes a -COOR₆ radical, R₆ being a lower alkyl radical, and in that the ester obtained is subjected to a hydrolysis for the purpose of obtaining the corresponding acid.

15. Process according to Claim 14, characterised in that the acid obtained is activated and then converted to the corresponding amide by the action of an amine of formula: 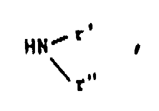 in which r' and r'' have the same meanings as those given in Claim 1.

16. Medicinal product, characterised in that it is a compound of formula (I) according to any one of Claims 1 to 12.

17. Pharmaceutical composition, characterised in that it contains at least one compound of formula (I) according to any one of Claims 1 to 12, in a vehicle suitable for enteral, parenteral, topical or ocular administration.

18. Composition according to Claim 17, characterised in that it is presented in a form suitable for topical application, and contains from 0.0001 to approximately 5 % by weight of a compound of formula (I).

19. Use of the compound according to any one of Claims 1 to 12 for the preparation of a pharmaceutical composition intended for the treatment of dermatological, rheumatic, respiratory and also ophthalmological conditions.

20. Cosmetic composition for body and hair hygiene, characterised in that it contains at least one compound or formula (I) according to any one of Claims 1 to 12, in a suitable cosmetic vehicle.

21. Cosmetic composition according to Claim 20, characterised in that it contains the compound of formula (I) at a concentration of between 0.0001 and 0.1 %, and preferably between 0.001 and 0.01 %, by weight.

## Patentansprüche

1. Aromatische Benzamidoverbindungen der folgenden allgemeinen Formel : 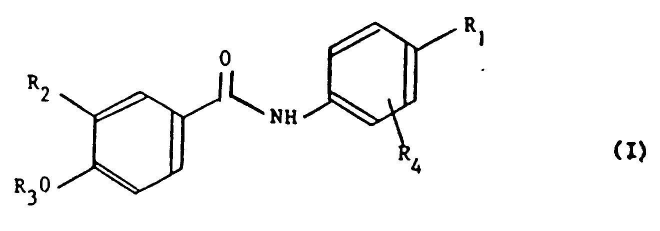 worin
R₁ für -CH₂OH, -CHOHCH₃ oder -COR₅ steht, wobei
R₅ für ein Wasserstoffatom, einen Niedrigalkylrest, den -OR₆-Rest oder 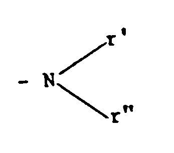 steht, wobei
R₆ ein Wasserstoffatom, einen Niedrigalkylrest oder einen Mono- oder Polyhydroxyalkylrest bedeutet, und
r' und r'' ein Wasserstoffatom, einen Niedrigalkylrest, einen Mono- oder Polyhydroxyalkylrest, einen gegebenenfalls substituierten Aryl- oder Benzylrest oder einen Aminosäure- oder Aminozuckerrest bedeuten, oder, zusammen genommen, einen Heterocyclus bilden,
R₂ für einen α,α'-disubstituierten Alkylrest mit 4 bis 12 Kohlenstoffatomen oder einen mono- oder polycyclischen Cycloalkylrest mit 5 bis 12 Kohlenstoffatomen steht, wobei das Bindungskohlenstoffatom quaternär ist,
R₃ für ein Wasserstoffatom oder einen Alkylrest mit 1 bis 10 Kohlenstoffatomen steht,
R₄ für ein Wasserstoffatom, einen Niedrigalkylrest oder einen Hydroxylrest steht,
mit Ausnahme einer Verbindung der Formel (I), worin R₁ für den Rest -CO₂CH₃ steht, R₂ für einen 1-Adamantylrest steht, R₃ für einen Decylrest steht und R₄ für einen Hydroxylrest steht, und die Salze der aromatischen Benzamidoderivate der Formel (I) , wenn R₆ ein Wasserstoffatom bedeutet.

2. Verbindungen nach Anspruch 1, wobei es sich bei den Verbindungen der Formel (I), wenn sie in Form von Salzen vorliegen, um die Alkali- oder Erdalkalimetallsalze, Zinksalze oder Salze mit einem organischen Amin handelt.

3. Verbindungen nach Anspruch 1, wobei der Niedrigalkylrest 1 bis 6 Kohlenstoffatome aufweist und ausgewählt ist unter einem Methyl-, Ethyl-, i-Propyl-, Butyl- und t-Butyl-Rest.

4. Verbindungen nach Anspruch 1, wobei der Monohydroxyalkylrest ein 2-Hydroxyethyl-, 2-Hydroxypropyl oder 3-Hydroxypropylrest ist.

5. Verbindungen nach Anspruch 1, wobei der Polyhydroxyalkylrest 3 bis 6 Kohlenstoffatome und 2 bis 5 Hydroxylgruppen aufweist und ausgewählt ist unter dem 2,3- Dihydroxypropyl-, 2,3,4-Trihydroxybutyl-, 2,3,4,5-Tetrahydroxypentyl- oder dem Pentaerythritolrest.

6. Verbindungen nach Anspruch 1, wobei der Arylrest ein gegebenenfalls mit einem Halogenatom, einer Hydroxyl- oder einer Nitrogruppe substituierter Phenylrest ist.

7. Verbindungen nach Anspruch 1, wobei der α,α'- disubstituierte Rest mit 4 bis 12 Kohlenstoffatomen ein t-Butyl- , 1,1-Dimethylpropyl-, 1-Methyl-1-ethylpropyl-, 1-Methyl-1-ethylhexyl- oder ein 1,1-Dimethyldecylrest ist.

8. Verbindungen nach Anspruch 1, wobei der mono- oder polycyclische Cycloalkylrest mit 5 bis 12 Kohlenstoffatomen , wobei der Bindungskohlenstoff quaternär ist, ein 1-Methylcyclohexyl- oder ein 1-Adamantylrest ist.

9. Verbindungen nach Anspruch 1, wobei der Aminosäurerest ein von Lysin oder Glycin abgeleiteter Rest ist.

10. Verbindungen nach Anspruch 1, wobei der Aminozuckerrest ein von Glucosamin, Galactosamin oder Mannosamin abgeleiteter Rest ist.

11. Verbindungen nach Anspruch 1, wobei die Reste r' und r'' zusammen einen Heterocyclus bilden, ausgewählt unter einem Piperidin-, Piperazin-, Morpholin-, Pyrrolidin- oder (2-Hydroxyethyl)-4-piperazinrest.

12. Verbindungen nach einem der vorhergehenden Ansprüche, ausgewählt unter:
- 4-[3-(1-Adamantyl)-4-methoxybenzamido]benzoesäure,
- 4-[3-(1-Adamantyl)-4-methoxybenzamido]methylbenzoat,
- N-Ethyl-4-[3-(1-adamantyl)-4-methoxybenzamido]-benzamid,
- 4-[3-(1-Adamantyl)-4-hydroxybenzamido]benzoesäure,
- 4-[3-(1-Adamantyl)-4-hydroxybenzamido]methylbenzoat,
- 4-[3-(1-methylcyclohexyl)-4-methoxybenzamido]benzoesäure,
- 4-[3-(1-Methylcyclohexyl)-4-methoxybenzamido]ethylbenzoat,
- 4-[3-(1-Adamantyl)-4-decyloxybenzamido]benzoesäure,
- 4-[3-(1-Adamantyl)-4-decyloxybenzamido]methylbenzoat,
- 4-[3-(1-Adamantyl)-4-hexyloxybenzamido]benzoesäure,
- 4-[3-(1-Adamantyl)-4-hexyloxybenzamido]methylbenzoat,
- 4-[3-(1,1-Dimethyldecyl)-4-methoxybenzamido]benzoesäure,
- 4-[3-(1,1-Dimethyldecyl)-4-methoxybenzamido]methylbenzoat,
- 4-(3-t-Butyl-4-methoxybenzamido)benzoesäure,
- 4-(3-t-Butyl-4-methoxybenzamido)methylbenzoat,
- N-[4-[3-(1-Adamantyl)-4-methoxybenzamido]-benzoyl]-pyrrolidin,
- N-[4-[3-(1-Adamantyl)-4-methoxybenzamido]-benzoyl]-piperidin,
- 4-[3-(1-Adamantyl)-4-methoxybenzamido]benzoesäuremorpholid,
- 4-[3-(1-Adamantyl)-4-methoxybenzamido]benzoesäure-t-butylamid,
- 4-[3-(1-Adamantyl)-4-methoxybenzamido]benzoesäureethylamid,
- 4-[3-(1-Adamantyl)-4-methoxybenzamido]benzoesäureanilid,
- 4-[3-(1-Adamantyl)-4-methoxybenzamido]benzoesäurebenzylamid,
- 4-[3-(1-Adamantyl)-4-methoxybenzamido]2-hydroxy ethylbenzoat,
- N-(4-Acetylphenyl)-3-(1-adamantyl)-4-methoxybenzamid,
- N-[4-(1-Hydroxyethyl)phenyl]-3-(1-adamantyl)-4-methoxybenzamid,
- 4-[3-(1-Adamantyl)-4-methoxybenzamido]benzoesäure-2-hydroxyethylamid,
- 2-Hydroxy-4-[3-(1-adamantyl)-4-methoxybenzamido]-methylbenzoat.

13. Verfahren zur Herstellung der Verbindungen nach einem der Ansprüche 1 bis 12, **dadurch gekennzeichnet,** daß in einem organischen Lösungsmittel und in Anwesenheit eines tertiären Amins eine aktivierte Form einer substituierten Benzoesäure, insbesondere ein Säurechlorid der Formel: 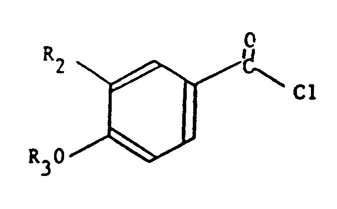 mit einer Aminoverbindung der Formel: 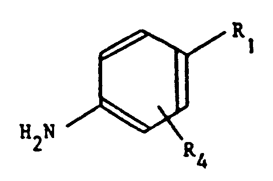 umgesetzt wird, wobei R₁, R₂, R₃ und R₄ die gleichen Bedeutungen wie in Anspruch 1 haben und die Reaktion bei Raumtemperatur unter Rühren durchgeführt wird.

14. Verfahren nach Anspruch 13, **dadurch gekennzeichnet,** daß R₁ einen -COOR₆-Rest bedeutet, wobei R₆ ein Niedrigalkylrest ist und der erhaltene Ester einer Hydrolyse unterzogen wird, wobei man die entsprechende Säure erhält.

15. Verfahren nach Anspruch 14, **dadurch gekennzeichnet,** daß die erhaltene Säure aktiviert und dann durch Einwirkung eines Amins der Formel: 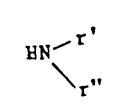 wobei
r' und r'' die gleichen Bedeutungen haben wie in Anspruch 1, zum entsprechenden Amid umgewandelt wird.

16. Medikament, **dadurch gekennzeichnet,** daß es sich dabei um eine Verbindung der Formel (I) nach einem der Ansprüche 1 bis 12 handelt.

17. Pharmazeutisches Mittel, **dadurch gekennzeichnet,** daß es zur enteralen, parenteralen, topischen oder okularen Verabreichung in einem geeigneten Träger mindestens eine Verbindung der Formel (I) nach einem der Ansprüche 1 bis 12 enthält.

18. Mittel nach Anspruch 17, **dadurch gekennzeichnet,** daß es in zur topischen Verabreichung geeigneter Form vorliegt und 0,0001 bis ca. 5 Gew.-% einer Verbindung der Formel (I) enthält.

19. Verwendung einer Verbindung nach einem der Ansprüche 1 bis 12 zur Herstellung eines zur Behandlung dermatologischer, rheumatischer, respiratorischer und ophthalmologischer Affektionen geeigneten pharmazeutischen Mittels.

20. Kosmetisches Mittel für die Hygiene des Körpers und der Haare, **dadurch gekennzeichnet,** daß es in einem geeigneten kosmetischen Träger mindestens eine Verbindung der Formel (I) nach einem der Ansprüche 1 bis 12 enthält.

21. Kosmetisches Mittel nach Anspruch 20, **dadurch gekennzeichnet,** daß es die Verbindung der Formel (I) in einer Konzentration von 0,0001 bis 0,1 Gew.-%, vorzugsweise von 0,001 bis 0,01 Gew.-%, enthält.
